# EUROPÄISCHE PATENTSCHRIFT

(11) **EP 1 188 422 B1**
(45) Veröffentlichungstag und Bekanntmachung des Hinweises auf die Patenterteilung: **03.05.2006**
(21) Anmeldenummer: 01118346.4
(22) Anmeldetag: 27.07.2001
(51) Int. Cl.: A61F 2/36, A61F 2/46

(54) **Schuhlöffelprothese**
Shoehorn prosthesis
Prothèse chausse-pieds

(30) Priorität: 29.07.2000 DE 10036985
(43) Veröffentlichungstag der Anmeldung: 20.03.2002
(73) Patentinhaber: Thurgauer Kantonalbank, 8570 Weinfelden (CH)
(72) Erfinder: Draenert, Klaus, 81545 München (DE); Walker, Norbert, 71706 Markgröningen (DE)
(74) Vertreter: Rentsch & Partner

(56) Entgegenhaltungen:
- EP-A- 0 496 089
- WO-A-90/02533
- DE-A- 4 234 351
- DE-C- 19 610 741
- FR-A- 2 716 107
- US-A- 4 129 903
- US-A- 5 571 203
- US-A- 5 658 333

## Beschreibung

### Hintergrund der Erfindung

Der schenkelhals des Oberschenkelknochens bildet anatomisch den stärksten Teil des menschlichen Skelettes. Aus diesem Grunde war es folgerichtig, diesen beim Gelenkersatz zu erhalten; das Ergebnis dieser Versuche waren die verschiedenen Ansätze, den Schenkelhalskopf zu überlappen (Wagner, H. Surface Replacement of the Hip. Clin Orthop 134:102-130,1978; Freeman, M. et al. Cemented double cup arthroplasty of the hip. Clin Orthop 134:45-52,1978). Die punktförmige Krafteinleitung unter der Kappe führte jedoch früh zu Schaukelbewegungen der Schale und zur Knochenresorption in der Folge der Retativbewegung bis zur Schenkelhalsfraktur. Es konnte aus histopathologischen Untersuchungen zu dieser Verankerung der Schluss gezogen werden, dass ein Steifigkeitssprung Implantat - Knochen nur in zentrifugaler Richtung von einem intramedullären Implantat auf den Knochen übertragen werden kann. Das Ziel dieser Erfindung ist daher die gleichmässige Krafteinleitung von einem steifen intramedullären Implantat auf den Knochen des Schenkelhalses.

### Stand der Forschung

Es fehlte nicht an Versuchen, im Schenkelhals Prothesenstiele zu verankern, so versuchten Huggler AH und Jacob HAC 1984 (Huggler AH and Jacob HAC The uncemented thrust plate prosthesis. In: Morscher E (Ed) The cementless fixation of hip endoprostheses. Berlin, Heidelberg, New York: Springer) mit der Druckscheibenprothese teilweise erfolgreich über eine Zuggurtungsverschraubung durch den Schenkelhals eine Prothesenstielkomponente zu verankern. Die Kragenauflage und die steife Konstruktion hatten jedoch Knochenatrophie und Knochenresorption zur Folge, was ein deutlicher Hinweis dafür war, dass die Krafteinleitung unphysiologisch war. Dies änderte sich auch nicht durch die Einführung hochporöser Strukturen im Stielelement (Patentschrift: DE 196 10 741 C1).
Die Zugankerprothese von Nguyen (Gold, T., Schill, S. und Menge,M. Die Zugankerprothese - 3 Jahre klinische Erfahrungen. Orthop Praxis 3: 194-197,1996) stellte eine Kombination von einer intramedullären Geradschaftprothese mit dem Zuggurtungsprinzip dar. Gerade die inneren Strukturen des Schenkelhalses erlauben jedoch nicht, gerade Schäfte zu verankern, sie erzwingen eine Spiegelsymmetrie zwischen einem rechten und linken Stiel. In histopathologischen Untersuchungen wurde nun gefunden, dass intramedulläre steife Kraftträger in gesetzmässig bestimmter Weise Kraft in die Knochenstrukturen einleiten, was in der nachfolgenden Erfindung folgerichtig umgesetzt worden ist.

Der Vorteil einer Prothese, die sich darauf beschränkt, die Kraft im Schenkelhals einzuleiten, ist der, dass im Falle eines unwahrscheinlichen Versagens der Verankerung eine normale Schaßverankerung ohne jeden Nachteil noch möglich ist.

### Beschreibung der Erfindung

Ein wesentliches Element des Prothesenstieles ist die gleichmässige Deformation der Schenkelhalsspongiosa und damit die Krafteinleitung in die Strukturen, die von der tragenden Gelenkfläche die Last aufnehmen und in die Knochenstrukturen des Schenkelhalses und der Femurdiaphyse einleiten, ohne die Deformation des Oberschenkelknochens als Ganzes zu behindern. Diese Strukturen lassen sich in dem so genannten U-shape umschreiben, welches im Schenkelhals dorsal, medial und ventral ausgebildet ist. Die Prothese hat dadurch einen U-förmigen Gelenkkörper, welcher die innere Oberfläche des Schenkelhalses vollständig auskleidet und innen hohl ist (U-shape: *Abb*. *02 und 03*).

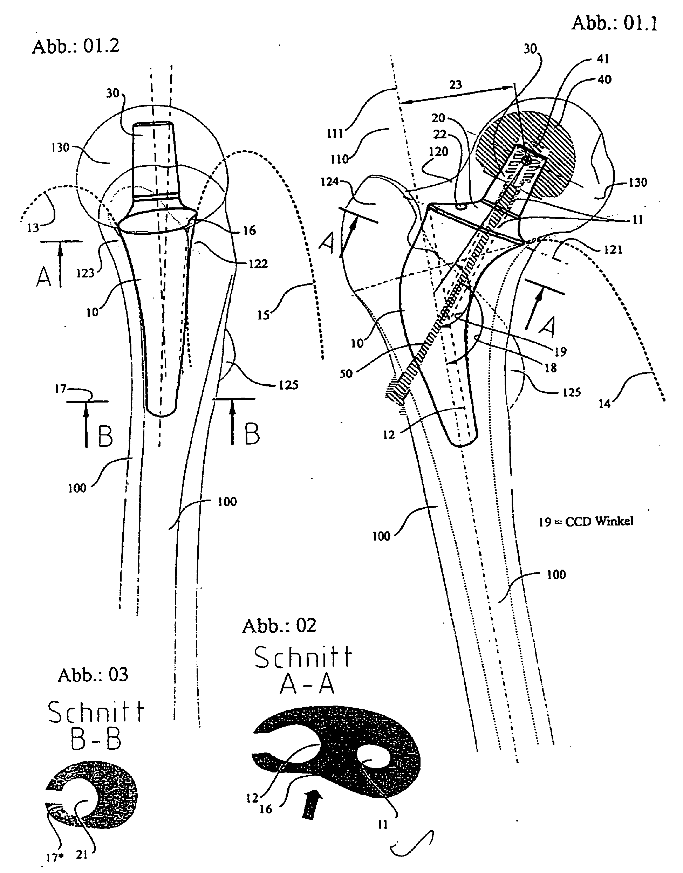

Die anatomische Schenkelhalsstruktur wird dabei vollständig erhalten, denn die Osteotomie verläuft vom lateralen Übergang des Schenkelhalses in das Trochantermassiv *(Abb*. *0.1.1*/*121*) bis zum medialen Kopf-Hals-Übergang; auf diese Weise bleiben die inneren Strukturen des Schenkelhalses *(Abb. 01.1*/*120)* vollständig erhalten. Die Prothese legt sich dorsal, medial und ventral der Wand des Femur anatomisch an und richtet sich im Femurkanal axial aus, sie überragt mit ihrer ventralen und medialen Mantelfläche parabelförmig *(Abb*. *01.1*/*13)* die Knochenstrukturen. Die Achse des Prothesenstieles *(Abb. 01.1*/*111)* ist in der Frontalebene durch ihren CCD - Winkel (Collum-Centrum-Diaphysenwinkel) zwischen 125° und 145°, in aller Regel 135°, und in der axialen Ebene des Schenkelhalses 5° bis 15°, in aller Regel 7°, definiert und projiziert sich über die Femurkanalachse *(Abb*. *01.1*/*111)*, parallel hierzu auch der lateral offene Hohlschaft *(Abb*. *01.1*/*12).* Die Prothese besteht aus dem kurzen Hohlschaft als Stiel *(Abb. 01.1*/*10),* der über eine Schulter *(Abb. 01.1*/*20)* in den Konus übergeht *(Abb. 01.1*/*30),* der wiederum als modulares System verschiedene Köpfe *(Abb*. *01.1*/*40)* zentrisch oder exzentrisch aufnehmen kann und welcher axial durchbohrt ist *(Abb*. *01.1*/*11)* für die Aufnahme einer Zuggurtungsvorrichtung Um dorsal die stabilen Schenkelhalsstrukturen zu erhalten, ist der Stiel coaxial tief eingezogen, wodurch der Querschnitt Nierenform erhält *(Abb. 02*/*A-A).*

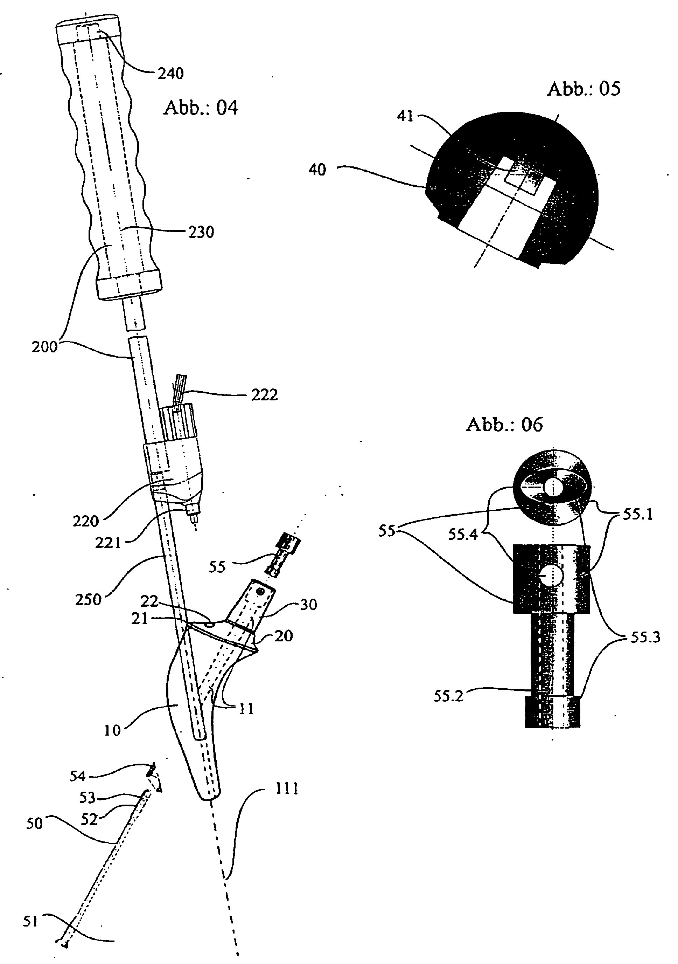

Die Neck-Slip Prothese wird mit dem führungsinstrument *(Abb*. *04*/*230)* axial eingesetzt. Die mediale Mantelfläche des Stieles *(Abb. 01.2*/*16)* und ventral *(Abb. 01.2*/*13)* wird die wandständige Spongiosa gleichmässig komprimiert. Das Führungsinstrument wird axial entlang der Prothesenachse eingeführt und mit der coaxial ausgerichteten fixationsvorrichtung *(Abb*. *04*/*220)* mit Gewinde *(Abb*. *04*/*221)* und Innenimbus *(Abb*. *04*/*222)* in die Probeprothese *(Abb*. *04*/*21)* aufgeschraubt. Die in dieser Richtung eingeschlagene Prothese wird nach Entfernen des Einschlaginstrumentes über dem Konus axial durch den Konuskanal *(Abb*. 0*4*/*11)* mit einem 4,5 mm Bohrer gebohrt. In der outside-in Technik wird der Zuganker *(Abb*. *06*/*55)* eingeschraubt und mit dem Drehmomentschlüssel bis auf 2,5 Nm angezogen. Der Probekopf *(Abb. 05*/*40)* ist so konstruiert, dass er nach Einsetzen der Mutter und nach Probereposition ein Zurückgleiten der Mutter verhindert, und zwar durch den Noppen *(Abb*. *05*/*41)*, welcher in den Konus mit axialem Bohrkanal *(Abb. 04*/*11)* und Kopfaufnahme *(Abb. 04*/*32)* eingreift.

### Beispiele der Erfindung

Das Hüftgelenk wird beispielsweise in Rückenlage des Patienten über einen Bauer' schen Zugang freigelegt und die Kapsel entfernt und der Femurkopf exartikuliert. Der Femurkopf wird unter Erhaltung des Schenkelhalses abgesetzt und die Pfanne präpariert, beispielsweise ein press fit-Pfannenimplantat eingeschliffen und ein Keramikinsert eingesetzt. Danach wird das Femur aussenrotiert und adduziert und der Markkanal mit der 11,2 mm Diamantschleife eröffnet. Der Kanal wird mit dem Führungsinstrument sondiert und wenn dieses ohne Widerstand eingeführt werden kann, ist die Achse des Femurkanals korrekt getroffen. Im Anschluss daran werden die Spongiosa der Metaphyse und die Spongiosa des Schenkelhalses soweit geschliffen, dass die Probeprothese eingesetzt werden kann, deren U-shape die innere Kortikalis des Schenkelhalses ventral und dorsal gerade eben überragt. Die Probeprothese wird herausgenommen und mit dem Applikator die korrespondierende Prothese eingeschlagen. Danach wird mit dem 4,5 mm Bohrer axial durch den Konus posterolateral durch die Femukompakta gebohrt und die Konusmutter in den Konus eingesetzt und der Probekopf "s" = small eingesetzt.

Das Bein wird anschliessend ohne Gewalt reponiert und in Normal-0-Stellung wird das Bohrloch aufgesucht und dann in Innenrotation und Abduktion die Zugankerlänge durch das Bohrloch mit der Messlehre gemessen, eingesetzt und angezogen mit einem Drehmoment von 2,5 Nm. Anschliessend wird das Gelenk luxiert, der Probekopf gegen den richtigen in der Länge passenden Kopf ausgetauscht, nachdem dieser durch Probereposition mit dem richtigen Probekopf definitiv ermittelt worden war.

Nach Reposition mit dem definitiven Keramikkopf der richtigen Länge wird unter Einlegen von Drainagen die Wunde verschlossen.
- *Abb. 01.1*: Neck Slip Prothese (NSP) in das Femur implantiert mit folgenden Merkmalen und anatomischen Korrelationen:
- *Abb. 01.1*/*10*: Sticl der NSP
- *Abb. 01.1*/*11*: Bohrkanal für den Schubanker
- *Abb. 01.1*/*12*: Coaxiale Begrenzung der Hohlrinne
- *Abb. 01.1*/*14*: Krümmung der medialen Mantelfläche
- *Abb. 01.1*/*18*: Antetorsionswinkel: Konus/Stiel
- *Abb. 01.1*/*19*: CCD-Winkel, Centrum-Collum-Diaphysenwinkel des Stieles
- *Abb. 01.1*/*20*: Prothesensehulter
- *Abb. 01.1*/*22*: Schraubgewinde für das Setzinstrument
- *Abb. 01.1*/*23*: Offset = RZ Stiel (Strecke)
- *Abb. 01.1*/*30*: Konus
- *Abb. 01.1*/*40*: Probekugel mit
- *Abb. 01.1*/*41*: Konusnoppen
- *Abb. 01.1*/*50*: Schubanker
- *Abb. 01.1*/*100*: Femurknochen -Diaphyse
- *Abb. 01.1*/*110*: Eröffnungsbohrung um die
- *Abb. 01.1*/*111*: Kanalachse
- *Abb. 01.1*/*120*: Schenkelhals und Metaphyse
- *Abb. 01.1*/*121*: Osteotomie an der Kopf-Hals-Grenze
- *Abb. 01.1*/*114*: Truchanter major
- *Abb. 01.1*/*125*: Trochanter minor
- *Abb. 01.1*/*130*: Schenkelhalskopf (Epiphyse)

- *Abb. 01.2*: Axialansicht der NSP
- *Abb. 01.2*/*13*: Krümmung der ventralen Mantelfläche des Stieles
- *Abb. 01.2*/*15*: Krümmung der dorsalen Mantelfläche des Stieles
- *Abb. 01.2*/*16*: dorsaler Niereneinschnitt
- *Abb. 01.2*/*122*: Calcar femoris, die hintere Schenkelhalswand
- *Abb. 01.2*/*123*: die vordere Schenkelhalswand

- *Abb. 02*: Querschnitt durch den proximalen Stiel mit den Merkmalen:
- *Abb. 01*/*12*: mediale, coaxiale Begrenzung der lateralen Hohlrinne
- *Abb. 02*/*16*: dorsale, nierenförmige Einziehung

- *Abb. 03*: Querschnitt oberhalb der Prothesenspitze
- *Abb. 03*/*17*: Schlüsselloch-Führung für das Setzinstrument um die Spitze
- *Abb. 03*/*11*: axiale Führungsrinne

- *Abb. 04*: Setzinstrument mit NSP-Prothese mit den folgenden Merkmalen:
- *Abb. 04*/*10*: Stiel der Prothese
- *Abb. 04*/*11*: Bohrkanal für den Zuganker
- *Abb. 04*/*20*: Schulter mit
- *Abs. 04*/*21*: Fühnmgsrinne für das axiale Setzinstrument
- *Abb. 04*/*22*: Gewinde für das
- *Abb.04*/*200*: Setzinstrument mit
- *Abb. 04*/*220*: Schraubenhalterung
- *Abs. 04*/*221*: Fixationsschraube
- *Abb, 04*/*222*: Innenimbus
- *Abb.04*/*230*: Griff
- *Abs.04*/*240*: Hammeraufschlag
- *Abb. 04*/*250*: Führungsstange

- *Abb. 04*/*30*: Konus mit
- *Abb. 04*/*11*: axialer Bohrung

- *Abb. 04*/*50*: Schubanker mit
- *Abb. 04*/*51*: Schraubenkopf
- *Abb. 04*/*52*: Gewinde
- *Abb. 04*/*53*: Kunsutoffsicherungen
- *Abb. 04*/*54*: Unterlegscheibe

- *Abb. 05*: Schnitt durch die Probekugel
- *Abb.05*/*40*: Probekopf mit
- *Abb. 05*/*41*: Konusnoppen zum Fixieren der Konusmutter

- *Abb. 06*: Aufsicht und Ansicht der Konusmutter
- *Abb. 06*/*55*: Konusmutter mit
- *Abb. 06*/*55.1*: Konusmutterkopf
- *Abb. 06*/*55.2*: Schaft
- *Abb. 06*/*55.3*: Axialzylinderführung
- *Abb. 06*/*55.4*: Koptbohrung
zum Herausnehmen der Mutter und innere Kopffräsung (ellipsoid) für die Aufnahme des verdrehsicheren Prohekopfes *(Abb. 05*/*40)* mit Noppen.

## Patentansprüche

1. Kurzschaft-Femurkomponente eines künstlichen Hüftgelenkes für die zementfreie Implantation, umfassend einen Schaft (10), eine Schulter (20), einen Konus (11) und einen Zuganker (50) zur Verankerung der Femurkomponente am Oberschenkelknochen **dadurch gekennzeichnet,**
- **dass** der Stiel der Prothese ohne Kragenauflage ausgeführt ist,
- der Stiel der Prothese dorsal eine konkave Einziehung aufweist, die axial geradlinig oder bogenförmig zur Spitze des Implantates ausläuft und Knochenstrukturen des Schenkelhalses an seinem Übergang zum grossen Trochanter aufnehmen kann,
- wodurch der Stiel der Prothese im Querschnitt Nierenform aufweist oder plump kommaförmig ist.

2. Kurzschaft-Femurkomponente nach Anspruch (1) so beschaffen, dass der Stiel lateral ganz oder teilweise offen ist.

3. Kurzschaft-Femurkomponente nach dem Anspruch (1) **dadurch gekennzeichnet, dass** der Zuganker in Richtung des Hüftkopfes verschieblich ist, jedoch unverschieblich in Zugrichtung.

4. Kurzschaft-Femurkomponente nach Anspruch (3) **dadurch gekennzeichnet, dass** der Zuganker drei Teile umfasst:
- eine Auflagenscheibe (54), die den Kopf der Schubstange am Knochen aufnehmen kann.
- eine Schubstange (50) bestehend aus Kopf mit Innenimbuss und endständigem Gewinde (53),
- eine Konusmutter (55) mit Gewinde und Innenimbus.

5. Kurzschaft-Fermurkomponente nach dem Anspruch (4) **dadurch gekennzeichnet, dass** die Konusmutter in ihrem Brett, im Konus der Prothese, verdrehgesichert ist, beispielsweise durch exzentrische Ausführung des Kopfes in seinem Bett.

6. Kurzschaft-Femurkomponente nach den Ansprüchen (4) oder (5) so beschaffen, dass der Zuganker an seinem endständigen Gewinde gegen die Konusmutter verdrehgesichert ist, beispielsweise durch 1bis 4 HDPE-Stopper.

7. Kurzschaft-Femurkomponente nach einem der Ansprüche (1) bis (6) **dadurch gekennzeichnet, dass** sie einen Probekopf (40) umfasst, der ein Zurückschieben der Konusmutter verhindert, beispielsweise durch einen zentralen Zapfen, der in den Konus hineingreift und an der Konusmutter aufsitzt und/oder in diese eingreift und so das Verdrehen verhindert.

8. Kurzschaft-Femurkomponente nach Anspruch (7), **dadurch gekennzeichnet, dass** der Probekopf einen zentralen Zapfen (41) besitzt.

9. Kurzschaft-Femurkomponente nach einem der Ansprüche (1) bis (8) **dadurch gekennzeichnet dass**, die Mantelfläche des Stieles in Umfangsrichtung ventral konvex und senkrecht dazu konkav (13) gekrümmt ist, in der Weise, dass der Flächenkrümmungsmittelpunkt ventral liegt und sich der Halbmesser nach proximal kontinuierlich verkleinert.

10. Kurzschaft-Femurkomponente nach den Ansprüchen (1) bis (9) so gestaltet, dass die mediale Mantelfläche des Stieles in Umfangsrichtung konvex und senkrecht dazu entlang der medialen Kontur konkav (14) gekrümmt ist und zwar in der Weise, dass der Mantelkrümmungsmittelpunkt medial liegt und sein Radius sich nach proximal kontinuierlich verkleinert (Parabel).

11. Femurkomponente nach einem der Ansprüche (1) bis (10) **dadurch gekennzeichnet, dass** die dorsale Mantelfläche des Stieles in Umfangsrichtung konkav oder konvex-konkav-konvex in der Form eines Wellenschlages oder einer gerundeten "3" mit asymmetrischen Schenkeln und gerundetem Übergang, und senkrecht dazu gerade ist oder konkav (15) gekrümmt ist mit dem Krümmungsmittelpunkt dorsal und einer kontinuierlichen Abnahme seines Radius gegen proximal.

12. Kurzschaft-Femurkomponente nach einem der Ansprüche (1) bis (11) **dadurch gekennzeichnet dass,** die Ventralfläche und/oder Medialfläche und/oder Dorsalfläche und/oder Lateralfläche des Stieles durch coaxial ausgerichtete Längswülste strukturiert ist /sind.

13. Kurzschaft-Femurkomponente nach einem do Ansprüche (1) bis (12) **dadurch gekennzeichnet, dass** der Stiel über eine Schulter in den Konus übergeht, der als modulares System verschiedene Köpfe zentrisch oder exzentrisch aufnehmen kann und der eine zentrale Bohrung aufweisen kann für die Aufnahme einer Zuggurtungsvorrichtung.

14. Kurzschaft-Femurkomponente nach einem der Ansprüche (1) bis (13) **dadurch gekennzeichnet, dass** der Konus zwischen 2° und 9°, in aller Regel um 4°-5°, in der Weise aufgerichtet ist, dass sich der CCD Winkel nicht verändert und auch das Offset unverändert bleibt, die Achse des Konus sich jedoch in die laterodorsale Zirkumefenz des kompakten Femur projiziert, und zwar 2cm bis 4cm unterhalb des tuberculum innominatum.

15. Kurzschaft-Femurkomponente nach einem der Ansprüche (1) bis (14) **dadurch gekennzeichnet, dass** sie als Werkstoff Titan, Tantal, CoCrMo oder eine Legierung aus Titan oder Tantal oder rostfreier Stahl enthält.

16. Kurzschaft-Femurkomponente nach einem der Ansprüche (1) bis (15) **dadurch gekennzeichnet, dass** ihre Oberfläche eine Reuhigkeit zwischen 70 und 250 µm, vorzugsweise 70 - 150 µm aufweist.

17. Kurzschaft-Femurkomponente nach emem der Ansprüche (1) bis (16), **dadurch gekennzeichnet, dass** sie weitere Zuganker umfasst, die über die Schulter und dorsal und ventral am Femur angebracht werden können.

18. Kurzschaft-Femurkomponente nach Anspruch 17, **dadurch gekennzeichnet, dass** die weiteren Zuganker Schubstangen und/oder Drahtseile oder Drahtzuggurtungen umfassen.

## Claims

1. A short-stem femur component of an artificial hip joint for the cement-free implantation, comprising a stem (10), a shoulder (20), a cone (11) and a tie rod (50) for anchoring the femur component on the femur, **characterised in that**
- the shank of the prosthesis is designed without a support collar,
- the shank of the prosthesis dorsally comprises a concave taper, which tapers axially in a straight line or in an arc-like manner to the tip of the implant, and may accommodate bone structures of the femoral neck at its transition to the large trochanter,
- by which means the shank of the prosthesis in cross section has a kidney shape or is plumply comma-shaped.

2. A short-stem femur component according to claim 1, **characterised in that** the shank laterally is completely or partly open.

3. A short-stem femur component according to claim 1, **characterised in that** the tie rod is displaceable in the direction of the head of the femur, but is undisplaceable in the tensile direction.

4. A short-stem femur component according to claim 3, **characterised in that** the tie rod comprises three parts:
- a support disk (54) which may accommodate the head of the push rod on the bone,
- a push rod (50) consisting of a head with an inner allen screw and a terminal thread (53),
- a cone nut (55) with a thread and inner allen screw.

5. A short-stem femur component according to claim 4, **characterised in that** the cone nut in its bed, in the cone of the prosthesis, is rotationally secured, for example by way of an eccentric design of the head in its bed.

6. A short-stem femur component according to one of the claims 4 or 5, **characterised in that** the tie rod at its terminal thread is rotationally secured against the cone nut, for example by way of 1 to 4 HDPE stoppers.

7. A short-stem femur component according to one of the claims 1 to 6, **characterised in that** it comprises a sample head (40) which prevents a sliding-back of the cone nut, for example by way of a central pin which engages into the cone and is seated on the cone nut and/or engages into this and thus prevents the rotation.

8. A short-stem femur component according to claim 7, **characterised in that** the sample head comprises a central pin (41).

9. A short-stem femur component according to one of the claims 1 to 8, **characterised in that** the peripheral surface of the shank in the peripheral direction is curved in a ventrally convex manner and concavely (13) perpendicular thereto, in a manner such that the surface curvature centre point lies ventrally and the radius reduces continually in the proximal direction.

10. A short-stem femur component according to the claims 1 to 9, **characterised in that** it is designed such that the medial peripheral surface of the shank in the peripheral direction is convexly curved and perpendicular to this along the medial contour is concavely (14) curved, and specifically in a manner such that the periphery curvature centre point lies medially and its radius reduces continuously in the proximal direction (parabola).

11. A femur component according to one of the claims 1 to 10, **characterised in that** the dorsal peripheral surface of the shank (3) is concave or convex-concave-convex in the shape of a wave runout or a rounded "3" with asymmetrical limbs and a rounded transition, and perpendicular to this is straight or concavely (15) curved with a dorsal curvature centre point, and with a continuous reduction of its radius in the proximal direction.

12. A short-stem femur component according to one of the claims 1 to 11, **characterised in that** the ventral surface and/or medial surface and/or dorsal surface and/or lateral surface of the shank is/are structured by coaxially aligned longitudinal beads.

13. A short-stem femur component according to one of the claims 1 to 12, **characterised in that** the shank via a shoulder merges into the cone, which as a modular system may accommodate different heads in a centric or eccentric manner, and which may comprise a central bore for accommodating a tension banding device.

14. A short-stem femur component according to one of the claims 1 to 13, **characterised in that** the cone is set up between 2° and 9°, as a rule by 4°-5°, in a manner such that the CCD angle remains unchanged or also the offset remains unchanged, the axis of the cone however projects into the laterodorsal circumference of the compact femur, and specifically 2 cm to 4 cm below the tuberculum innominatum

15. A short-stem femur component according to one of the claims 1 to 14, **characterised in that** as a material it contains titanium, tantalum, CoCrMo or an alloy of titanium or tantalum or stainless steel.

16. A short-stem femur component according to one of the clam 1 to 15, **characterised in that** its surface has a roughness between 70 and 250 µm, preferably 70 - 150 µm.

17. A short-stem femur component according to one of the claims 1 to 16, **characterised in that** it comprises further tie rods which may be attached via the shoulder and dorsally and ventrally on the femur.

18. A short-stem femur component according to claim 17, **characterised in that** the further tie rods comprise push rods and/or wire cables and/or wire tension banding devices.

## Revendications

1. Composant de fémur à tige ou broche courte d'une articulation de hanche artificielle pour l'implantation sans ciment, comprenant une tige ou broche (10), un épaulement (20), un cône (11) et un(e) ancre de traction ou tirant (50) pour ancrer le composant de fémur sur ou dans l'os de la cuisse, **caractérisé en ce que**
- le pédicule de la prothèse est réalisé sans support de collerette,
- le pédicule de la prothèse comporte sur sa face dorsale un retrait concave qui se termine axialement en ligne droite ou en forme d'arc en direction de la pointe de l'implant et peut accueillir des structures osseuses du col du fémur à sa transition avec le grand trochanter,
- le pédicule de la prothèse étant ainsi en forme de rein ou grossièrement en forme de virgule dans sa section transversale.

2. Composant de fémur à tige courte selon la revendication 1, de forme telle que le pédicule est entièrement ou partiellement ouvert latéralement.

3. Composant de fémur à tige courte selon la revendication 1, **caractérisé en ce que** l'ancre de traction peut être décalée ou déplacée en direction de la tête de la hanche, mais ne peut pas être décalée ou déplacée dans le sens de traction.

4. Composant de fémur à tige courte selon la revendication 3, **caractérisé en ce que** l'ancre de traction ou tirant comprend trois parties :
- une plaque d'appui (54) qui peut accueillir la tête de la barre à coulisse ou de poussée au niveau de l'os,
- une barre à coulisse ou de poussée (50) composée d'une tête à pans creux internes et filetage terminal (53),
- un écrou conique (55) avec filetage et pans creux internes.

5. Composant de fémur à tige courte selon la revendication 4, **caractérisé en ce que** l'écrou conique est protégé contre la rotation dans son lit, dans le cône de la prothèse, par exemple grâce à une réalisation excentrée de la tête dans son lit.

6. Composant de fémur à tige courte selon les revendications 4 ou 5, configuré de telle manière que l'ancre de traction ou tirant est protégé(e) contre la rotation par rapport à l'écrou conique sur son filetage terminal, par exemple grâce à 1 à 4 stoppeurs ou arrêts en PEhd.

7. Composant de fémur à tige courte selon une des revendications 1 à 6, **caractérisé en ce qu'**il comprend une tête d'essai ou de sonde (40) qui empêche un recul de l'écrou conique, par exemple grâce à un pivot central qui pénètre dans le cône et est placé sur l'écrou conique et/ou entre en prise avec ce dernier, empêchant ainsi la rotation.

8. Composant de fémur à tige courte selon la revendication (7), **caractérisé en ce que** la tête d'essai possède un pivot central (41).

9. Composant de fémur à tige courte selon une des revendications 1 à 8, **caractérisé en ce que** la surface d'enveloppe du pédicule est courbée de manière convexe ventrale dans la direction de la circonférence ou périphériquement et de manière concave (13) perpendiculairement à cette dernière, de telle manière que le point central de la courbure d'enveloppe est ventral et que le rayon affiche une diminution proximale continue.

10. Composant de fémur à tige courte selon les revendications 1 à 9, configuré de telle manière que la surface d'enveloppe médiale du pédicule est convexe dans la direction de la circonférence ou périphériquement et concave (14) perpendiculairement à cette dernière le long du contour médial et ce, de telle manière que le point central de courbure d'enveloppe est médial et que son rayon affiche une diminution proximale continue (parabole).

11. Composant de fémur selon une des revendications 1 à 10, **caractérisé en ce que** la surface d'enveloppe dorsale du pédicule est courbée de manière concave ou convexe-concave-convexe sous la forme d'une vague ou d'un « 3 » arrondi avec des branches asymétriques et une transition arrondie et est droite ou concave (15) dans la direction perpendiculaire, avec le point central de courbure dorsal et une diminution proximale continue de son rayon.

12. Composant de fémur à tige courte selon une des revendications 1 à 11, **caractérisé en ce que** la surface ventrale et/ou la surface médiale et/ou la surface dorsale et/ou la surface latérale du pédicule est/sont structurée(s) par des bourrelets longitudinaux à orientation coaxiale.

13. Composant de fémur à tige courte selon une des revendications 1 à 12, **caractérisé en ce que** le pédicule passe, via un épaulement, au cône, qui peut en tant que système modulaire accueillir de manière centrée ou excentrée différentes têtes et comporter un perçage central pour le logement d'un dispositif de bandage par suspension ou traction.

14. Composant de fémur à tige courte selon une des revendications 1 à 13, **caractérisé en ce que** le cône est dressé entre 2° et 9°, généralement d'environ 4° à 5°, de telle manière que l'angle CCD ne se modifie pas et que l'offset reste également identique, mais que l'axe du cône se projette cependant dans la circonférence latérodorsale du fémur compact et ce, 2 à 4 cm en dessous du tuberculum innominatum.

15. Composant de fémur à tige courte selon une des revendications 1 à 14, **caractérisé en ce qu'**il contient comme matériau du titane, du tantale, du CoCrMo ou un alliage de titane, de tantale ou d'acier inoxydable.

16. Composant de fémur à tige courte selon une des revendications 1 à 15, **caractérisé en ce que** sa surface affiche une rugosité de 70 à 250 µm, de préférence de 70 à 150 µm.

17. Composant de fémur à tige courte selon une des revendications 1 à 16, **caractérisé en ce qu'**il comprend d'autres ancres de traction ou tirant qui peuvent être posé(e)s ou arrangé(e)s sur l'épaulement et sur les faces dorsale et ventrale du fémur.

18. Composant de fémur à tige courte selon la revendication 17, **caractérisé en ce que** les autres ancres de traction ou tirants comprennent des barres coulissantes et/ou des fils d'acier ou bien des bandages par traction ou suspension en fil d'acier.
